# EUROPÄISCHE PATENTSCHRIFT

(11) **EP 0 994 144 B1**
(45) Veröffentlichungstag und Bekanntmachung des Hinweises auf die Patenterteilung: **08.12.2004**
(21) Anmeldenummer: 99119586.8
(22) Anmeldetag: 02.10.1999
(51) Int. Cl.: C08G 77/455, C08H 1/00

(54) **Polypeptid-Polysiloxan-Copolymere**
Polypeptid-polysiloxane copolymers
Copolymères à base de polypeptides et de polysiloxanes

(30) Priorität: 17.10.1998 DE 19848002
(43) Veröffentlichungstag der Anmeldung: 19.04.2000
(73) Patentinhaber: Goldschmidt AG, 45127 Essen (DE)
(72) Erfinder: Dietz, Thomas, Dr., 45259 Essen (DE); Lersch, Peter, Dr., 46119 Oberhausen (DE); Weitemeyer, Christian, Dr., 45134 Essen (DE)

(56) Entgegenhaltungen:
- EP-A- 0 540 357
- EP-A- 0 699 431
- EP-A- 0 736 297
- US-A- 3 562 353
- PATENT ABSTRACTS OF JAPAN vol. 017, no. 339 (C-1075), 28. Juni 1993 (1993-06-28) & JP 05 039359 A (KAO CORP), 19. Februar 1993 (1993-02-19)
- PATENT ABSTRACTS OF JAPAN vol. 1995, no. 06, 31. Juli 1995 (1995-07-31) & JP 07 070204 A (LION CORP), 14. März 1995 (1995-03-14)
- CHARLES M. KANIA: "Preparation of Poly(dimethylsiloxane)-Polypeptide Block Copolymers." JOURNAL OF APPLIED POLYMER SCIENCE, Bd. 27, Nr. 1, 1982, Seiten 139-148, XP002167314

## Beschreibung

Die vorliegende Erfindung betrifft Polypeptid-Polysiloxan-Copolymere, deren Herstellung durch thermische Copolymerisation von Aminosäuren mit organofunktionellen Polysiloxanen sowie deren Verwendung als grenzflächenaktive Substanzen.

Proteine sind natürlich vorkommende Polypeptide und spielen eine wichtige Rolle in allen biologischen Prozessen. Sie werden in zunehmendem Maße in Körperpflegemitteln als Konditionierer, Feuchthaltemittel und Weichmacher eingesetzt. Proteine sind natürliche, hochmolekulare Polymere und werden im allgemeinen zu niedermolekularen Proteinen hydrolysiert, damit sie wasserlöslich werden. Zwar lassen sich Proteinhydrolysate leichter in Formulierungen einarbeiten, allerdings sind die löslichen Proteine weniger substantiv auf Haut und Haar.

Silicon ist der Sammelbegriff für eine Vielzahl von Verbindungen mit unterschiedlichen Eigenschaften, die alle jedoch durch die Silicium-Sauerstoff-Bindung in der Siloxankette charakterisiert sind. Sie spielen wie Proteine ebenfalls eine wichtige Rolle in der Kosmetik, insbesondere als Konditioniermittel. Polydimethylsiloxane beispielsweise sind substantiv auf Haut und Haar, verleihen dem Haar Glanz und Glätte und erzeugen auf der Haut ein angenehm weiches und seidenes Gefühl. Allerdings sind sie praktisch nicht in Wasser löslich. Mit Siliconpolyethern hat man zwar wasserlösliche Siliconderivate, die auf Haar Glätte erzeugen können, allerdings sind diese nur wenig substantiv.

Proteine und Silicone stellen somit zwei sehr unterschiedliche Substanzklassen mit ebenfalls unterschiedlichen Eigenschaften und Vorteilen dar, die jeweils für kosmetische Anwendungen wertvoll sind. Durch die Entwicklung von Proteinen, die auch einige charakteristische Eigenschaften von Siliconen besitzen wie die Glättung von Haut und Haar oder von Siliconen, die Vorzüge von Proteinen wie höhere Wasserlöslichkeit und Substantivität besitzen, erhält man Zugang zu Produkten mit Eigenschaften, die nicht durch einfache Mischungen aus beiden Substanzklassen erhalten werden können.

Bereits in US-A-3 562 353 wird die Kombination von Siliconen mit Polypeptiden in Form von Copolymeren beschrieben. Dabei handelt es sich um Blockcopolymere vom ABA- oder (AB)n-Typ, die durch Kupplung endfunktionalisierter Homopolymere erhalten werden. A ist ein Polyamidteil mit einer Molekularmasse von 2 000 bis 100 000 und B ein Siliconteil mit einer Molekularmasse von 500 bis 100 000. Bei den Verbindungen handelt es sich um thermoplastische Blockcopolymere, die entweder elastisch oder fest sind und als biokompatible Implantatmaterialien verwendet werden können. Diese werden hergestellt durch Reaktion eines aus alpha-Aminosäuren bestehenden Polyamids mit reaktiven Endgruppen wie Hydroxyalkyl-, Aminoalkyl- oder Isocyanatogruppen mit einem Silicon, das reaktive Endgruppen wie Chloralkyl-, Carbonsäure-, Isocyanato-, Hydroxyalkyl - oder Aminoalkylgruppen trägt. Das funktionelle Polyamid muß jedoch erst mit großem synthetischen Aufwand unter Einbezug von Schutzgruppen-Chemie hergestellt werden. In einer ersten Stufe wird aus der alpha-Aminosäure durch Umsetzung mit Phosgen in einem Lösungsmittel wie Dioxan das entsprechende N-Carboxyanhydrid hergestellt. Handelt es sich bei der alpha-Aminosäure um eine Dicarbonsäure wie Glutaminsäure oder Asparaginsäure, so muß zunächst die eine Carboxylgruppe durch Veresterung beispielsweise mit einem Überschuß an Benzylalkohol in Anwesenheit von Bromwasserstoffsäure verestert werden. Handelt es sich um eine alpha-Aminosäure mit einer weiteren Amino-, Hydroxy- oder Mercaptogruppe, so müssen diese ebenfalls vor der Reaktion mit Phosgen in geeigneter Weise geschützt werden, um unerwünschte Nebenreaktionen zu vermeiden. In einer zweiten Stufe wird die geschützte alpha-Aminosäure dann zum Polyamid umgesetzt. Diese mehrstufige Synthese sei an folgendem Beispiel näher erläutert: Geht man von dem als Benzylester geschützten N-Carboxyanhydrid der L-Glutaminsäure, dem sogenannten N-Carboxy-gamma-benzyl-L-glutamat, aus, so wird dieses mit Ethanolamin als Initiator in Dimethylformamid als Lösungsmittel polymerisiert. Nach etwa 90 % Umsatz muß das N-Carboxyanhydrid von Phenylalanin zugegeben werden, damit es die Endgruppe des Polyamids bildet. Das gebildete Polymer muß mit Wasser ausgefällt und mit Methanol gewaschen werden. Im nächsten Schritt wird das Polyamid in epsilon-Caprolacton als Reagenz und Lösungsmittel über 50 h (!) erhitzt, erneut mit Wasser ausgefällt und mit Methanol gewaschen. Auf diese Weise erhält man ein Polyamid, das an beiden Enden Hydroxyalkylgruppen trägt. Das dihydroxyfunktionelle Polyamid wird dann in einer Mischung aus Benzol und Dichlorbenzol als Lösungsmittel mit alpha, omega-bis(Dimethylamino)-poly(dimethylsiloxan) unter Abspaltung von Dimethylamin umgesetzt. Das Polymer wird mit Methanol ausgefällt und mit Hexan gewaschen. Zur Herstellung der in US-A-3 562 353 beschriebenen Copolymere ist also eine Vielzahl von Reaktions- und Aufarbeitungsschritten erforderlich, einschließlich aufwendiger Schutzgruppenchemie. Ferner werden zum Teil sehr giftige Reagenzien wie Phosgen benötigt und die Reaktionen werden in Lösungsmitteln wie Benzol und Dimethylformamid durchgeführt, von denen das Produkt wiederum befreit werden muß. In der Regel enthält der Polypeptidteil Schutzgruppen enthaltende Aminosäuren wie Benzylglutaminsäure und unpolare Aminosäuren wie Phenylalanin. Damit sind die Copolymere praktisch wasserunlöslich. Auf der anderen Seite erfolgt die Verknüpfung zwischen Polyamid- und Siliconteil durch eine hydrolyseempfindliche Si-O-C-Bindung, so daß bei einer Entfernung der Schutzgruppen die Bindung zwischen Silicon- und Peptidteil wieder gespalten würde und zusätzlich Abbaureaktionen am Polysiloxan ausgelöst würden.

In Journal of Applied Polymer Science, 27, 1982, 139 - 148 wird ebenfalls die Herstellung von Polypeptid-Polysiloxan-Blockcopolymeren beschrieben. Diese werden erhalten durch Polymerisation der N-Carboxyanhydride von Phenylalanin und gamma-Benzylglutaminsäure mit einem alpha, omega-aminopropylfunktionellen Polydimethylsiloxan als Initiator. Bei den erhaltenen Blockcopolymeren handelt es sich um weiße, weiche Feststoffe. Wie jedoch schon in US-A-3 562 353 werden zur Herstellung der Copolymere eine Vielzahl von Reaktions- und Aufarbeitungschritten sowie Schutzgruppen und Lösungsmittel benötigt. Eine typische Reaktionsdauer der Polymerisation liegt im Bereich zwischen 100 - 200 h (!).

In der US-A-5 100 956 werden Silicon-Protein-Copolymere beansprucht, in denen der Siliconteil über eine Polyetherphosphatgruppe mit der Aminogruppe eines Proteins verknüpft ist. Durch die Polyetherphosphateinheit sind die Polymere zwar wasserlöslich, allerdings besitzen sie auch eine sehr hydrolyseempfindliche Phosphorsäureesterfunktion, so daß der Silicon- und Proteinteil wieder leicht voneinander abgespalten werden können. Außerdem ist anzunehmen, daß die Polyetherreste, die als Abstandshalter und Verknüpfungselement zwischen Protein- und Siliconteil wirken, aufgrund ihrer Polymerenverteilung und des damit einhergehenden hochmolekularen Charakters die Eigenschaften der Produkte nicht unbeeinflußt lassen und eher das Eigenschaftsbild von Hybrid-Silicon-Polyether-Protein-Copolymeren aufweisen, als daß sie als reine Silicon-Protein-Copolymere wirken. Die Silicon-Protein-Copolymere werden hergestellt, indem wasserlösliche epoxyfunktionelle Polysiloxane mit Hydrolysaten natürlicher Proteine in Wasser umgesetzt werden. Die Wasserlöslichkeit der Polysiloxane wird dabei durch hydrosilylierende Anlagerungen von Polyethern und anschließender Phosphatierung der Hydroxygruppe erreicht. Die zur Reaktion mit freien Aminogruppen des Proteins fähige Epoxygruppe wird anschließend durch Reaktion des Natriumsalzes des Siliconphosphats mit Epichlorhydrin in das Silicon eingeführt. Somit beinhaltet auch dieser Syntheseweg mehrere Stufen sowie die Verwendung gefährlicher und hochgiftiger Reagenzien wie Phosphorpentoxid oder Epichlorhydrin.

In einem weiteren US-Patent 5 243 028 wird dann auch eine verbesserte Verfahrensvariante zur Herstellung von Silicon-Protein-Copolymeren beschrieben. Hierbei wird zunächst ein hydroxyfunktioneller Siliconpolyether mit Chloressigsäure zum entsprechenden chloressigsäureesterfunktionellen Siloxan umgesetzt. Daran anschließend erfolgt die Umsetzung mit Proteinen bzw. Proteinhydrolysaten unter definierten Bedingungen, wobei im Rahmen einer Substitutionsreaktion das organisch gebundene Chlor in die Chloridform übergeführt wird und die Verknüpfung mit dem Protein erfolgt. Obwohl dieses Verfahren insgesamt eine Verbesserung darstellt, kann aber auch hier nicht auf die Verwendung von ätzender und giftiger Chloressigsäure verzichtet werden. Ebenfalls nachteilig ist, daß die Verknüpfung zwischen Siliconrücken und dem Proteinrest über eine nicht hydrolysestabile Estergruppe erfolgt, was die Verwendung derartiger Materialien in wäßrigen Formulierungen stark einschränkt und eine wäßrige Langzeitlagerung sogar unmöglich macht. Weiterhin ist zu befürchten, daß derartige Produkte aufgrund der hygroskopischen Eigenschaften des Proteinrestes selbst in fester Form nur unzureichend stabil sind und daß mit fortschreitender Lagerzeit eine zunehmende Rückspaltung in Siliconpolyether und freies Protein stattfindet. Werden, wie in den Beispielen beschrieben, Siliconpolyether als Ausgangsmaterialen verwendet, sind auch hier die Produkte keine echten Silicon-Proteine, sondern weisen einen erheblichen Hybridcharakter auf.

In EP-A-0 540 357 (Croda, GB 9 123 251, Nov. 1991) werden Protein-Silicon-Copolymere beansprucht, in denen die Siliconkomponente kovalent mit den Aminogruppen des Proteins verknüpft ist und in jedem Fall zumindest einige der Siliconkomponenten zur Vernetzung zwischen verschiedenen Protein-Ketten beitragen, zusätzlich aber auch nicht vernetzende Siloxaneinheiten enthalten sein können. Als Proteinkomponente dienen natürliche Proteine wie Kollagen, Elastin u.a., die entweder partiell hydrolysiert oder durch chemische Modifizierung wie Veresterung oder Quaternisierung modifiziert wurden. Die Copolymere entstehen durch Reaktion funktioneller Gruppen von Silanen oder Siliconen mit den Aminogruppen des Proteins. Dadurch entstehen höhermolekulare Polymere, die auch miteinander vernetzte Proteinketten enthalten. Eine zusätzliche Vernetzung kann durch die Kondensation von Silanolgruppen der Silane oder Silicone erfolgen. Eine wesentliche Voraussetzung für die Reaktion der Proteinkomponente ist deren Löslichkeit in Wasser oder einem anderen geeigneten Lösungsmittel wie Ethanol oder Propylenglykol oder in Mischungen aus beiden. Eine weitere Voraussetzung ist die Fähigkeit der Siliconkomponente, eine Vernetzung mit der Proteinkomponente zu bewirken. Dazu benötigt man entweder polyfunktionelle Silicone mit geeigneten reaktiven Gruppen wie Säurehalogenid-, Anhydrid- oder Epoxidgruppen oder monofunktionelle Siliciumverbindungen, die Silanolgruppen oder Gruppen, aus denen sich durch Hydrolyse in situ Silanolgruppen bilden können, enthalten, die durch Kondensation zu Siloxanbindungen eine Vernetzung herbeiführen. Damit die Siliciumverbindung mit dem Protein reagieren kann, muß es in demselben Lösungsmittel wie das Protein, bei dem es sich vorzugsweise um ein wäßriges Proteinhydrolysat handelt, löslich sein. In Wasser als Lösungsmittel wird deshalb ein organofunktionelles Silan mit hydrolysierbaren Gruppen benötigt. Dabei müssen die Reaktionsbedingungen sehr sorgfältig kontrolliert werden. Denn zum einen ist in der Regel ein pH-Wert oberhalb von 7 erforderlich, damit die Aminogruppen des Proteins reaktiv sind. Zum anderen findet im Alkalischen in der Regel eine rasche Hydrolyse der abspaltbaren Gruppen statt. Gleichzeitig erfolgt aber auch die Kondensation des Silans, so daß die Gesamtreaktion nur schwer zu steuern ist. Auf diese Weise erhält man daher nur vernetzte Produkte. Da in solchen Produkten keine linearen Polydimethylsiloxan-Segmente vorhanden sind, sind auch ihre typischen Siliconeigenschaften nur wenig ausgeprägt. Die Produkte sind zudem nur in Form wäßriger Lösungen handhabbar, da ein harter, wasserunlöslicher Film entsteht, sobald man das Wasser durch Destillation oder Trocknen entfernt. Damit die Reaktion beispielsweise in Ethanol durchgeführt werden kann und somit auch organofunktionelle Dimethylsilicone eingesetzt werden können, die in Wasser nicht, zumindest aber zu geringen Anteilen in Ethanol löslich sind, muß man Ethylester des Proteinhydrolysats einsetzen, was wiederum zusätzliche Reaktionsschritte beinhaltet. Ferner wird zur Einstellung des für die Reaktion benötigten pH-Wertes Natriumhydroxid benötigt, das bei Reaktionstemperaturen um 70 °C einen unerwünschten Siloxankettenabbau verursachen kann. Es wird angeführt, daß die chemische Struktur der Protein-Silicon-Copolymere sehr komplex ist und es daher nicht möglich ist, ihnen eine einzelne allgemeine Strukurformel zuzuordnen.

In EP-A-0 699 431 werden silylierte Peptide beansprucht, in denen die Aminogruppe eines Peptids nur eine Silylgruppe trägt. Die Verknüpfung zwischen Siliciumverbindung und Peptid wird auf ähnliche Weise wie in EP-A-0 540 357 durch Reaktion der Aminogruppen des Peptids mit einer reaktiven Gruppe der Siliciumverbindung hergestellt.

Als Siliciumverbindung werden Silane mit einer Halogenalkylgruppe eingesetzt. Damit die hydrophilen Peptide mit den hydrophoben Silylverbindungen in Wasser reagieren können, müssen zunächst die übrigen Gruppen des Silans hydrolysiert werden, damit das Silan wasserlöslich wird. Bei der Verwendung von Halogenalkylsilanen entsteht eine Halogenwasserstoffsäure, die den pH-Wert der Reaktionsmischung erniedrigt. Aus diesem Grund muß der pH-Wert der Reaktionsmischung durch Zugabe von Natriumhydroxid konstant gehalten werden, damit die Reaktion der Halogengruppe mit Wasser vermieden wird. Damit pro Peptid mindestens zwei Silylgruppen eingeführt werden können, muß das Peptid Aminosäuren mit einer zusätzlichen Aminogruppe, wie es bei Lysin der Fall ist, enthalten. Der Siliciumanteil wird also nur in Form von Silylgruppen, und zwar von nur einer Silylgruppe pro Aminogruppe des Proteins eingeführt. Aufgrund dessen ist auch bei den silanbasierenden Protein-Silicon-Copolymeren wie in EP-A-0 540 357 nicht mit einem Dimethylsiliconeffekt zu rechnen.

Natürliche Proteine und synthetische Peptide sind lineare Polymere aus Aminosäuren, die über eine Amidbindung (Peptidbindung) miteinander verknüpft sind. Beim Erhitzen einer Aminosäure auf über 100 °C erhält man jedoch nicht üblicherweise ein Polymer, sondern beobachtet eine rasche Schwarzverfärbung, was unter anderem auf die Bildung von Heterocyclen zurückzuführen ist. Ausnahmen hierzu sind Asparaginsäure, die beim Erhitzen Polysuccinimid bildet, das unter basischen Bedingungen in Polyasparaginsäure übergeführt werden kann. Glutaminsäure cyclisiert beim Erhitzen zu der monomeren Pyroglutaminsäure (2-Pyrrolidon-5-carbonsäure). In den frühen 50er Jahren entdeckten Fox und Middlebrook (Chemtech, Mai 1996, S. 26 - 29), daß man beim Erhitzen von Glutaminsäure und Asparaginsäure ein Copolymer aus beiden Aminosäuren erhält. Auch andere Aminosäuren, die allein nicht zur Bildung von Polymeren befähigt sind, können mit Glutaminsäure und/oder Asparaginsäure zu Copolymeren umgesetzt werden. Eine Besonderheit dieser thermischen Proteine" bzw. Proteinoide" ist, daß sie eine nicht-statistische Verteilung in der Aminosäuresequenz besitzen. Aus dieser Beobachtung hat sich eine eigenständige Forschungsrichtung entwickelt, die auf dem Ursprung des Lebens auf Basis solcher, unter terrestrischen Bedingungen erzeugbaren Proteine beruht. Thermische Proteine sind aufgrund ihrer im Vergleich zu natürlichen Proteinen niedrigeren Molekularmasse von bis zu 9 000 nicht toxisch und somit mit lebenden Systemen biokompatibel. So finden sie zum Beispiel Anwendung bei der Mikroverkapselung von Pharmazeutika (US-A-4 963 364, US-A-4 925 673), als künstliche Haut (US-A-4 996 292) oder als Wirkstoff zur Verbesserung der Gedächtnisleistung (US-A-5 373 085). Auch die industrielle Anwendung als Inhibitoren der Mineralablagerung in Kühlwassersystemen wird beschrieben (US-A-4 534 881). Ein weiterer wichtiger Vorteil ist ihre biologische Abbaubarkeit.

Die Diskussion des Standes der Technik zeigt, daß Silicon-Protein-Copolymere bekannt sind, diese aber bislang gravierende Nachteile aufweisen. Entweder sind die Copolymere wasserunlöslich, da die Peptidkomponente Schutzgruppen tragende Aminosäureeinheiten enthält, oder sie sind wasserlöslich, besitzen jedoch dann eine hydrolyseempfindliche Bindung zwischen Peptidund Siliconteil. Auch die bekannten Verfahren zur Herstellung solcher Silicon-Protein-Copolymere weisen erhebliche Nachteile auf. Entweder handelt es sich um aufwendige, mehrstufige Herstellverfahren, bei denen oftmals giftige Substanzen benötigt werden, oder um einfache Verfahren, wie die Silylierung von Peptiden. Von den Produkten kann jedoch kein echter Siliconeffekt erwartet werden.

Die Aufgabe der Erfindung bestand nun darin, neuartige Silicon-Peptid-Copolymere zu finden, die wasserlöslich und gleichzeitig hochmolekular und somit substantiv sind. Ferner sollten sie längere Poly(dimethylsiloxy)-Einheiten enthalten und somit einen deutlichen Siliconeffekt aufweisen. Zum anderen galt es, ein Verfahren zu finden, das einfach in der Durchführung ist und keine giftigen Reagenzien benötigt.

Es wurde nun überraschend gefunden, daß durch thermische Copolymerisation von natürlichen und ungeschützten Aminosäuren, insbesondere Asparaginsäure und Glutaminsäure mit organofunktionellen Polysiloxanen Polypeptid-Polysiloxan-Copolymere erhalten werden können, die in eine wasserlösliche Form überführt werden könnten und dabei jedoch einen echten Siliconeffekt zeigen.

Die chemische Kombination solcher thermischer Proteine mit Siliconen zu Silicon-Protein-Copolymeren ist nicht bekannt. Es wurde überraschend gefunden, daß trotz der drastischen Reaktionsbedinungen wie Temperaturen von über 170 °C in einer pH-sauren Aminosäureschmelze reaktive Organopolysiloxane während der thermischen Polymerisation von insbesondere Asparaginsäure und Glutaminsäure und weiterer Aminosäuren in das Peptid unter Erhalt der Dimethylsiliconketten eingebaut werden können.

Gegenstand der Erfindung sind somit neuartige Polypeptid-Polysiloxan-Copolymere, Verfahren zu deren Herstellung und deren Verwendung als grenzflächenaktive Substanzen.

Gegenstand der vorliegenden Erfindung sind in einer ersten Ausführungsform Polypeptid-Polysiloxan-Copolymere, bestehend aus wenigstens einer Polysiloxaneinheit 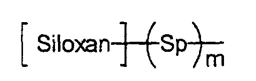 wobei der Index m eine positive ganze Zahl im Bereich von m = 1 - 52 darstellt,
der allgemeinen durchschnittlichen Formel I: 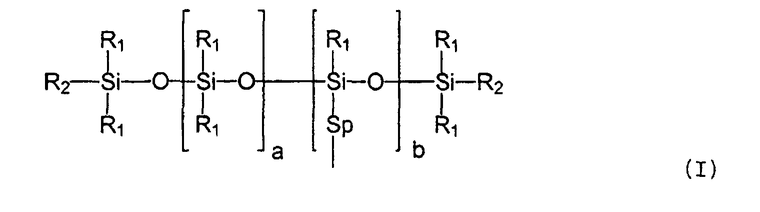 mit
R₁ = Alkylrest mit 1 bis 4 C-Atomen,
R₂= R₁ und/oder―Sp ―
wobei
― Sp ― = divalenter Abstandshalter (Spacer) zwischen Siloxan und einer weiteren funktionellen Gruppe ist, wobei Siliciumatom und Abstandshalter über eine Silicium-Kohlenstoff-Bindung verknüpft sind, insbesondere ein divalenter Alkylenrest mit 1 bis 20 C-Atomen, der gegebenenfalls verzweigt ist, Doppelbindungen oder aromatische Ringe sowie Heteroatome, insbesondere Sauerstoff, Stickstoff oder Schwefel enthält,
die Indices a und b ganze Zahlen in den Bereichen von a = 0 - 200 und b = 0 - 50 darstellen,
mit der Maßgabe, daß für a = b = 0 und für b = 0 und a ≠ 0 jeweils wenigstens ein R₂ = - Sp - ist
und aus wenigstens einer Polypeptideinheit 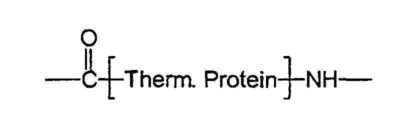 wobei
Therm. Protein für eine Struktur
der allgemeinen durchschnittlichen Formel II: 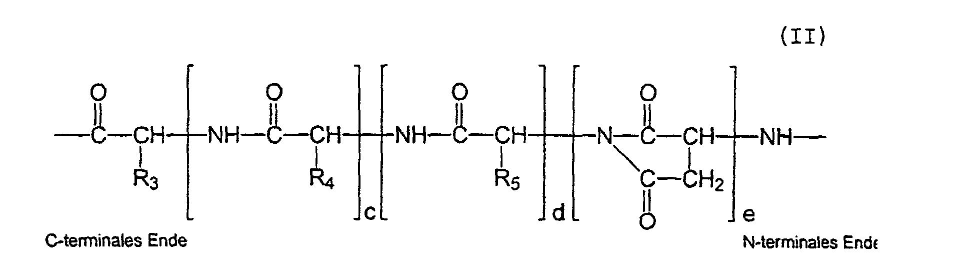 oder der Formel III: 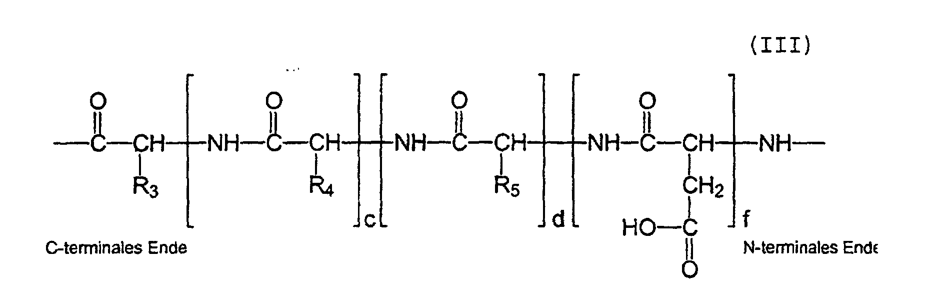 steht,
die über eine divalente funktionelle Gruppe
- FG -
entweder über das C-terminale, das N-terminale Ende oder beide Enden der Polypeptideinheit mit der Polysiloxaneinheit verknüpft ist und für eine Struktureinheit - CH(OH)CH₂- oder -CH(OH)CH₂O-, -CO-, -CH(CH₂CO₂H)CO-, -NH-, -O-, -S-,-CH(NH₂)CO- oder -CH(CO₂H)NH- steht und gegebenenfalls über die Reste R₄ und/oder R₅ zusätzliche Verknüpfungen zwischen Polysiloxan- und Polypeptideinheiten erfolgen
mit
R₃ = R₄ oder R₅,
wobei
R₄ = gleich einem Rest einer Aminosäure sowie -(CH₂)₄-NH-R₆ ist,
mit R₆ = H (Lysin) oder 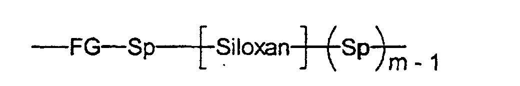 R₅ = -CH₂-CH₂-CO-R₆
mit R₆ = OH (Glutaminsäure) oder 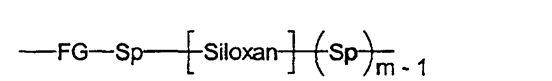 c, d, e und f positive ganze Zahlen einschließlich 0 sind,
mit den Maßgaben, daß
die Indices c, d, e in Formel II und c, d und f in Formel III nicht sämtlich 0 sind,
insbesondere e ≠ 0, wenn c = 0 ist,
c und d ≠ 0 sind, wenn e oder f = 0 ist
sowie
die Molekularmasse der Polypeptideinheit zwischen 250 und 9 000 liegt
und das Gewichtsverhältnis von Polysiloxaneinheiten und Polypeptideinheiten im Polypeptid-Polysiloxan-Copolymer zwischen 1 : 99 und 99 : 1 liegt.

Beispiele erfindungsgemäßer Verbindungen sind:
A) 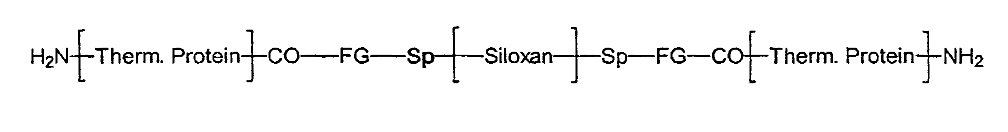 worin 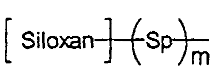 (m = 2) gleichbedeutend einer Polysiloxaneinheit folgender Struktur ist: 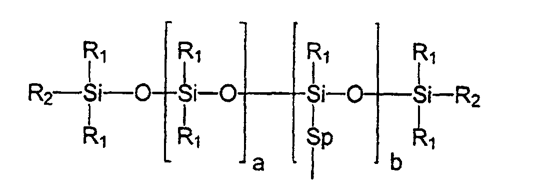 mit
   R₁ = CH₃
   beide R₂ = - Sp -
   Sp = - (CH₂)₃ -,
   a = 8
   b = 0
   und 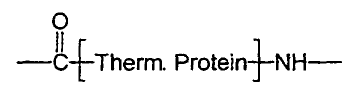 gleichbedeutend einer Polypeptideinheit folgender Struktur ist: 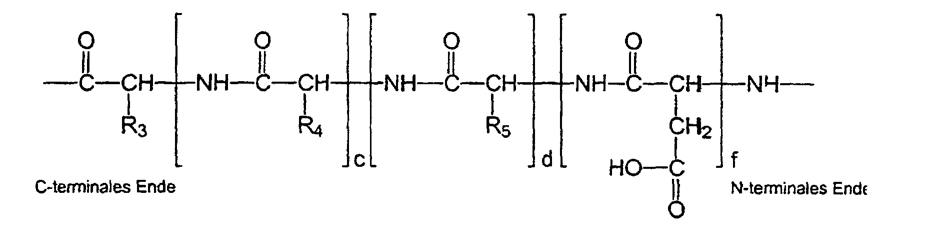 mit R₃ = - CH₂CO₂H oder (CH₂)₂CO₂H
   c = 0
   d und f ≠ 0 sind und das Verhältnis d : f ungefähr = 1 : 6 ist,
   FG = - NH -,
   die Molekularmasse der Polypeptideinheit ca. 500 beträgt und das Gewichtsverhältnis von Polysiloxan- zu Polypeptideinheiten im Copolymer 1 : 9 beträgt.
B) 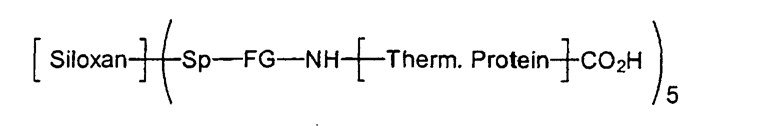 wobei die Polysiloxan- und die Polypeptideinheiten den in Beispiel A) angeführten Strukturen entsprechen mit
   R₁ = CH₃
   R₂ = R₁
   - Sp - = ― (CH₂)₃ - O - CH₂ -
   a = 20
   b = 5
   und
   mit R₃ = ― CH₂CO₂H oder - (CH₂)₂CO₂H
   c = 0
   d und f ≠ 0 sind und das Verhältnis d : f ungefähr = 1 : 6 ist,
   FG = - CH(OH)CH₂-,
   die Molekularmasse der Polypeptideinheit ca. 2 000 beträgt und das Gewichtsverhältnis von Polysiloxan- zu Polypeptideinheiten im Copolymer 3 : 7 beträgt.
C) 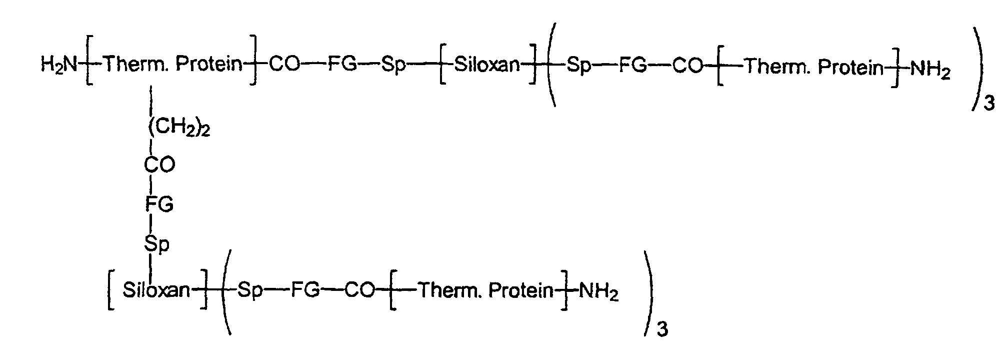 wobei Polysiloxan- und Polypeptideinheiten den in Beispiel A) angeführten Strukturen entsprechen mit
   R₁ = CH₃
   beide R₂ = - Sp-
   - Sp - = - (CH₂)₃-,
   a = 40
   b = 2
   und
   mit R₃ = - CH₂CO₂H oder - (CH₂)₂CO₂H
   c ≠ 0 und R₄ = - CH₂SH (Cystein)
   d und f ≠ 0 sind und das Verhältnis d : f ungefähr = 1 : 4 ist,
   FG = -NH-,
   der Gewichtsanteil von Cystein am Polypeptidteil ca. 5 %, die Molekularmasse der Polypeptideinheit ca. 1 500 und das Gewichtsverhältnis von Polysiloxan- zu Polypeptideinheiten im Copolymer 4 : 6 beträgt.
D) 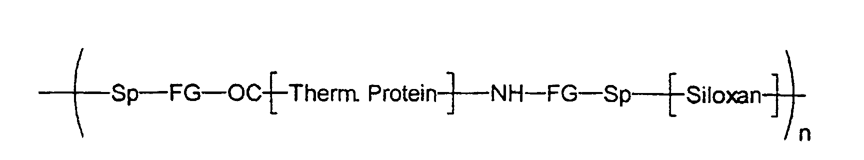 wobei die Polysiloxaneinheit der in Beispiel A) angeführten Struktur entspricht mit
   R₁ = CH₃
   beide R₂ = - Sp -
   - Sp - = - (CH₂)₃ - O - CH₂ - CH(OH) - CH₂ - NH - (CH₂)₄ -
   a = 18
   b = 0
   und die Polypeptideinheit folgender Struktur entspricht: 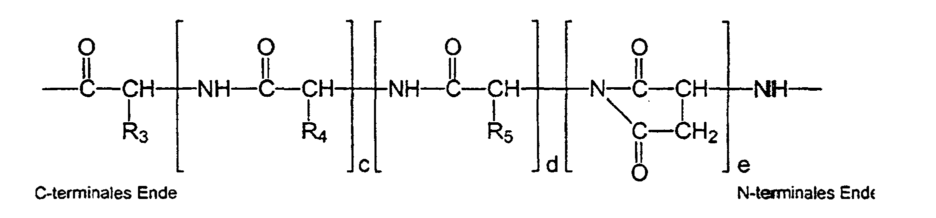 mit R₃ = - CH₂CO₂H
   c und d = 0
   e ≠ 0 ist
   FG = - CH(NH₂)CO - oder - CH(CO₂H)NH -,
   die Molekularmasse der Polypeptideinheit ca. 1 000 und das Gewichtsverhältnis von Polysiloxan- zu Polypeptideinheiten im Copolymer 1 : 9 beträgt.

Die Besonderheit dieser erfindungsgemäßen neuen Verbindungsklasse besteht darin, daß sie in einem einfachen Verfahren und ohne Verwendung von Schutzgruppen oder Lösungsmitteln erhalten werden kann. Ein besonderer Vorteil besteht darin, daß man von definierten Verbindungen, nämlich (natürlichen) Aminosäuren und organisch modifizierten Polysiloxanen ausgeht. Dies steht im Gegensatz zu solchen Verfahren, die von Proteinhydrolysaten ausgehen, die sich je nach Quelle des Proteins (tierisch oder pflanzlich), Herstellungsprozeß (pH-Wert, Reaktionstemperatur, -dauer) und der Lagerdauer der Lösung stark voneinander unterscheiden können. Eine Reproduzierbarkeit der Produktqualität ist somit nur schwer zu gewährleisten.

Ein weiterer wesentlicher Vorteil der vorliegenden Verbindungsklasse ist, daß deren tensidische Eigenschaften gezielt und maßgeschneidert in reproduzierbarer Weise eingestellt werden können. Dies wird in einfacher Weise durch die Wahl der Ausgangsverbindungen und deren Gewichtsverhältnis erreicht. Durch die Wahl des Gewichtsverhältnisses von Aminosäuren zu Polysiloxan, üblicherweise zwischen 95 : 5 und 40 : 60, wird der Anteil an Polydimethylsiloxaneinheiten im wesentlichen vorbestimmt, was wiederum die tensidischen Eigenschaften gravierend beeinflußt. Ein weiterer Parameter ist die Struktur des Polysiloxans. Es ist offensichtlich, daß die Anordnung und die Anzahl der funktionellen Gruppen im Polysiloxan einen großen Einfluß auf die Eigenschaften des Copolymers ausübt. Das Siloxan kann funktionelle Gruppen an beiden Enden der Kette oder seitenständig in unterschiedlicher Anzahl tragen. Es macht einen Unterschied in den Produkteigenschaften, welche Kettenlänge beispielsweise ein endfunktionalisiertes Polysiloxan hat bzw. welche Kettenlänge und wieviele funktionelle Gruppen pro Kette ein kammartiges Polysiloxan besitzt. Eine weitere Möglichkeit zur Modifizierung der tensidischen Eigenschaften ist die Art und das Verhältnis der eingesetzten Aminosäuren untereinander. So kann beispielsweise durch Zusatz hydrophober Aminosäuren wie Phenylalanin die Hydrophilie des Polypeptidteils reduziert werden. Weiterhin kann das Molekulargewicht des Copolymers durch die Reaktionsführung eingestellt werden, insbesondere durch die Temperatur und die Dauer des Erhitzens.

Entscheidend ist jedoch, daß im Gegensatz zu den aus der Literatur bekannten Protein-Polysiloxan-Copolymeren die Verbindungen Poly(dimethylsiloxy)-Ketten enthalten, mit denen als hydrophober Teil zusammen mit dem hydrophilen Polypeptidteil durch eine echte chemische Verknüpfung ein Tensid gebildet und zum anderen ein echter Silicon-Effekt erzielt wird. Ein weiterer Vorteil ist, daß die Copolymere, je nach Form der Aufarbeitung, in wasserunlöslicher oder wasserlöslicher Form erhalten werden können. In wasserunlöslicher Form können sie beispielsweise in unpolare Medien eingebracht werden. Sie können aber auch in wasserlöslicher Form als wäßrige Lösungen oder - nach Entfernen des Wassers - in fester Form erhalten werden. Dabei handelt es sich um ein trockenes, leicht rieselfähiges Pulver, das sich in Wasser in jedem Verhältnis in Form klarer Lösungen mischt. Durch Destillation des Wassers kann es aus diesen Lösungen zurückerhalten werden. Damit unterscheidet es sich deutlich von Proteinhydrolysat-Organosilan- oder Organosiloxan-Lösungen wie in EP-A-0 540 357 beschrieben, die nach Entfernung des Wassers einen harten Film bilden, der sich nicht mehr in Wasser löst.

Ein weiterer Gegenstand der vorliegenden Erfindung ist ein Verfahren zur Herstellung der oben beschriebenen Polypeptid-Polysiloxan-Copolymere durch thermische Polymerisation von Aminosäuren der allgemeinen Formel: 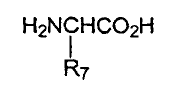 wobei R₇ gleich oder verschieden der Rest einer Aminosäure wie Glycin, Alanin, Valin, Leucin, Isoleucin, Phenylalanin, Prolin, Serin, Threonin, Tyrosin, Asparagin, Glutamin, Arginin, Lysin, Tryptophan, Histidin, Cystein, Methionin, Asparaginsäure, Glutaminsäure ist, in Anwesenheit von Organopolysiloxanen mit reaktiven Gruppen - RG der oben definierten Formel (I).

Die einzusetzenden Organopolysiloxane sind aus dem Stand der Technik bekannt und sind kommerziell erhältlich oder können leicht in an sich bekannter Weise hergestellt werden. Endständig epoxy- oder aminofunktionalisierte Polysiloxane erhält man z. B. durch Hydrosilylierung von Allylglycidylether oder Allylamin an ein endständig funktionalisiertes Wasserstoffsiloxan. Kammartige Aminopropylsiloxane werden z. B. durch Kondensation und alkalische Äquilibrierung von Aminopropyldialkoxysilanen und Siloxancylen hergestellt.

Beispiele für geeignete organofunktionelle Polysiloxane sind:

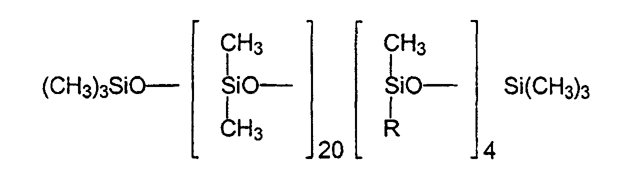

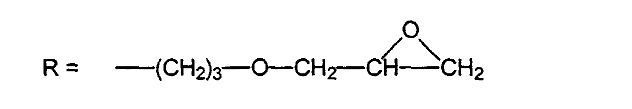

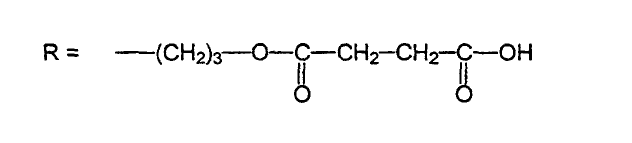

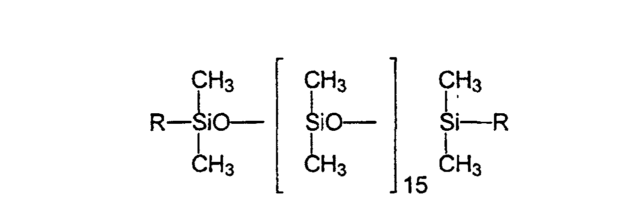

R = ―(CH₂)₃―NH₂

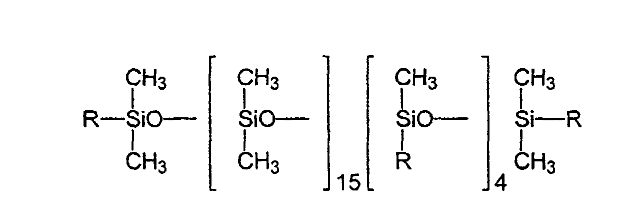

R = ―(CH₂)₆―OH

Das Herstellungsverfahren sei exemplarisch im folgenden beschrieben. In einer ersten Stufe wird beispielsweise Glutaminsäure bei ca. 170 - 180 °C geschmolzen, wobei sich durch Abspaltung von Wasser das cyclische Amid, die Pyroglutaminsäure (2-Pyrrolidon-5-carbonsäure), bildet. Statt der gebildeten Pyroglutaminsäure kann man auch Prolin oder ein polares, hochsiedendes Lösungsmittel wie N-Methylpyrrolidon oder Sulfolan verwenden. Man gibt dann Asparaginsäure zu und erhitzt die Schmelze oder die hochsiedende Lösung auf 160 - 220 °C. Dabei bildet sich Polysuccinimid, das im Fall der Verwendung von Glutaminsäure auch Glutaminsäureeinheiten enthält. Durch die Dauer und die Temperatur des Erhitzens wird die Molekularmasse des wachsenden Polymers gesteuert. Je länger die Dauer des Erhitzens und je höher die Temperatur, desto größer ist die Molekularmasse. Im nächsten Schritt tropft man das organofunktionelle Polysiloxan zu. Die Dauer des Erhitzens nach vollständiger Zugabe des Polysiloxans beeinflußt wiederum die Molekularmasse des gebildeten Copolymers. Man gießt die Schmelze aus und erhält nach Abkühlen eine glasartige Masse, die sich leicht durch Mörsern pulverisieren läßt. Dies ist die wasserunlösliche Form des Polypeptid-Polysiloxan-Copolymers.

Die wasserlösliche Form des Polypeptid-Polysiloxan-Copolymers erhält man durch Behandlung des Copolymers mit alkalischer wäßriger Lösung, beispielsweise mit wäßriger Natronlauge. Wird dabei der Neutralpunkt überschritten, so kann mit beispielsweise wäßriger Salzsäure neutralisiert werden. Die so erhaltene wäßrige Lösung des Copolymers kann entweder direkt verwendet werden oder man destilliert das Wasser ab und erhält ein wasserlösliches Pulver.

In einer Verfahrensvariante kühlt man zunächst die Pyroglutaminsäureschmelze auf ca. 120 °C ab, gibt dann zunächst das organofunktionelle Polysiloxan zu, erhöht nach einiger Zeit die Temperatur auf 170 °C und gibt dann erst die Asparaginsäure zu. Diese Verfahrensvariante hat sich insbesondere bei kammartigen Polysiloxanen als vorteilhaft erwiesen.

Ferner ist Gegenstand der vorliegenden Erfindung die Verwendung der Polypeptid-Polysiloxan-Copolymere in tensidischen Anwendungen, insbesondere als Silicontenside.

Die erfindungsgemäßen Polypeptid-Polysiloxan-Copolymere können in vielfältigen Anwendungen eingesetzt werden. Sie eignen sich besonders zum Einsatz in wäßrigen Medien, aus denen sie aufgrund ihrer Grenzflächenaktivität und ihrer Affinität zu Oberflächen ihre Wirkung entfalten. In Abhängigkeit von ihrem Aufbau können sie beim Einsatz in Kunststoffen die Oberflächenbeschaffenheit verbessern. Sie können als Öl-in-Wasser- oder Wasser-in-Öl-Emulgatoren oder als Stabilisatoren in Emulsionen Einsatz finden oder beispielsweise in kosmetischen Präparaten zur Reinigung von Haut und Haaren, zur Verbesserung des Schaumes und zur Konditionierung der Haare und/oder zur Erzielung eines angenehmen Hautgefühls eingesetzt werden. Als Proteinderivate können sie als Hautbefeuchtungsmittel (Moisturizer) oder als Mittel zur Linderung von Reizzuständen der Haut eingesetzt werden. Naturgemäß werden die erfindungsgemäßen Polypeptid-Polysiloxan-Copolymere häufig zusammen mit Tensiden und anderen Additiven zur Beeinflussung der Oberflächenbeschaffenheit eingesetzt. Alle genannten Formulierungen können bekannte Zusatzstoffe enthalten, wie beispielsweise Netzmittel, Tenside oder Emulgatoren aus den Klassen der anionischen, kationischen, zwitterionischen, amphoteren oder nichtionogenen oberflächenaktiven Substanzen, beispielsweise Fettalkoholsulfate, Fettalkoholethersulfate, Alkylsulfonate, Alkylbenzolsulfonate, Sulfobernsteinsäurealkylester, quartäre Ammoniumsalze, Alkylbetaine, Carbonsäureamidoalkylbetaine, Derivate von monomeren oder höherkondensierten Sacchariden, oxethylierte Fettalkohole, Fettsäurealkanolamide oder oxethylierte Fettsäureester, Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl.

Ferner ist ein Einsatz der erfindungsgemäßen Verbindungen als Textilhilfsmittel oder als Additiv in Farben und Lacken möglich.

Die folgenden Beispiele veranschaulichen die vorliegende Erfindung.

### Beispiele:

### Beispiel 1

In einem 250-ml-Dreihalskolben, ausgestattet mit elektrischem Heizmantel, Rührer, Tropftrichter und Thermometer, wurden 50 g Glutaminsäure unter Überleiten von Stickstoff für eine halbe Stunde auf 180 °C erhitzt. Bei der Reaktionsapparatur handelte es sich um eine offene Apparatur, so daß das während der Reaktion entstandene Wasser durch den Stickstoffstrom zum großen Teil entfernt wurde. Man stellte anschließend die Temperatur der Schmelze auf 170 °C ein und gab während einer halben Stunde portionsweise 50 g Asparaginsäure zu. Man hielt die Temperatur eine weitere halbe Stunde bei 170 °C und ließ dann während einer halben Stunde 66,5 g (40 Gew.-% bezogen auf Gesamtansatz) eines α,ω-endständigen Aminopropyldimethylpolydimethylsiloxans (X-22-161 AS von Shin Etsu) zutropfen. Anschließend erhitzte man noch eine Stunde auf 170 °C. Man goß die noch heiße Schmelze in einen Mörser aus. Nachdem die Schmelze zu einer glasartigen Masse erstarrt war, wurde sie möglichst fein gemörsert. Das gelbe Pulver rührte man 12 h in 1,5 l Wasser, filtrierte den unlöslichen Rückstand ab, wusch den Rückstand mit Wasser und Ethanol und trocknete anschließend im Trockenschrank bei 60 °C. Man erhielt 95 g (57 % Ausbeute) eines gelben, in Wasser unlöslichen Pulvers. Das Produkt konnte zur NMR-spektroskopischen Charakterisierung in [D6] Dimethylsulfoxid gelöst werden.

Das gelbe Pulver wurde zunächst mit 1 l 0,1 N Natronlauge, dann mit 630 ml 1 N Natronlauge gerührt, bis eine fast klare Lösung entstand. Man neutralisierte mit 3 N wäßriger Salzsäure und destillierte anschließend das Wasser bei 100 °C im Ölpumpenvakuum ab. Man erhielt ein gelbes Pulver, das in beliebiger Konzentration in Wasser löslich war.

### Beispiele 2 - 5/Vergleichsbeispiel:

In den Beispielen 2 bis 5 wurde der Anteil des Aminosiloxans am Gesamtansatz gemäß Beispiel 1 variiert. Die Versuchsdurchführung blieb gleich. Als Vergleich wurde ein nicht erfindungsgemäßes Beispiel herangezogen.

### Beispiel 2:

11 g (10 Gew.-% bezogen auf Gesamtansatz) Aminopropyldimethylpolydimethylsiloxan (X-22-161 AS von Shin Etsu)

### Beispiel 3:

25 g (20 Gew.-%) Aminopropyldimethylpolydimethylsiloxan

### Beispiel 4:

42,8 g (30 Gew.-%) Aminopropyldimethylpolydimethylsiloxan

### Beispiel 5:

100 g (50 Gew.-%) Aminopropyldimethylpolydimethylsiloxan

### Vergleichsbeispiel:

0 g (0 Gew.-%) Aminopropyldimethylpolydimethylsiloxan

### Beispiel 6:

In einem 250-ml-Dreihalskolben, ausgestattet mit elektrischem Heizmantel, Rührer, Tropftrichter und Thermometer, wurden 50 g Glutaminsäure unter Überleiten von Stickstoff für eine halbe Stunde auf 180 °C erhitzt. Bei der Reaktionsapparatur handelte es sich um eine offene Apparatur, so daß das während der Reaktion entstandene Wasser durch den Stickstoffstrom zum großen Teil entfernt wurde. Man stellte anschließend die Temperatur der Schmelze auf 170 °C ein und gab während einer halben Stunde portionsweise 75 g einer Mischung aus Asparaginsäure und Cystein (2 : 1) zu. Man hielt die Temperatur eine weitere halbe Stunde bei 170 °C und ließ dann während einer halben Stunde 83 g (40 Gew.-% bezogen auf Gesamtansatz) eines α,ω-endständigen Aminopropyldimethylpolydimethylsiloxans (X-22-161 AS von Shin Etsu) zutropfen. Anschließend erhitzte man noch eine Stunde auf 170 °C. Man goß die noch heiße Schmelze in einen Mörser aus. Nachdem die Schmelze zu einer glasartigen Masse erstarrt war, wurde sie möglichst fein gemörsert. Das gelbe Pulver rührte man 12 h in 1,5 l Wasser, filtrierte den unlöslichen Rückstand ab, wusch den Rückstand mit Wasser und Ethanol und trocknete anschließend im Trockenschrank bei 60 °C. Man erhielt 129 g (62 % Ausbeute) eines gelben, in Wasser unlöslichen Pulvers. Das Produkt konnte zur NMR-spektroskopischen Charakterisierung in [D6]Dimethylsulfoxid gelöst werden.

Das gelbe Pulver wurde zunächst mit 1 l 0,1 N Natronlauge, dann mit 900 ml 1 N Natronlauge gerührt, bis eine fast klare Lösung entstand. Man neutralisierte mit 3 N wäßriger Salzsäure und destillierte anschließend das Wasser bei 100 °C im Ölpumpenvakuum ab. Man erhielt ein gelbes Pulver, das in beliebiger Konzentration in Wasser löslich war. Der Schwefelgehalt wurde zu 1,1 % bestimmt.

### Beispiel 7:

In einem 250-ml-Dreihalskolben, ausgestattet mit elektrischem Heizmantel, Rührer, Tropftrichter und Thermometer, wurden 50 g Glutaminsäure unter Überleiten von Stickstoff für eine halbe Stunde auf 180 °C erhitzt. Bei der Reaktionsapparatur handelte es sich um eine offene Apparatur, so daß das während der Reaktion entstandene Wasser durch den Stickstoffstrom zum großen Teil entfernt wurde. Man kühlte die Schmelze auf 120 °C ab und tropfte während einer halben Stunde 66,5 g (40 Gew.-% bezogen auf Gesamtansatz) eines α,ω-endständigen epoxyfunktionellen Polydimethylsiloxans (DMS-E12 von Gelest) zu. Man erhitzte eine weitere Stunde auf 150 °C, erhöhte dann die Temperatur auf 170 °C und gab dann während einer halben Stunde 50 g Asparaginsäure zu. Nach 1 h bei 170 °C kühlte man auf 100 °C ab, gab 83 g 1 N Natronlauge zu und ließ unter Rühren weiter abkühlen. Man gab soviel festes Natriumhydroxid zu, bis sich der Feststoff aufgelöst hatte. War der pH-Wert der wäßrigen Lösung alkalisch, so wurde anschließend mit 3 N wäßriger Salzsäure neutralisiert. Das Wasser wurde im Ölpumpenvakuum weitgehend abdestilliert, die gelbe und etwas klebrige Masse wurde dann im Trockenschrank bei 60 °C getrocknet. Man erhielt ein gelbes Pulver, dessen 1 %ige wäßrige Lösung opak war und sehr gut schäumte.

### Beispiel 8:

In einem 250-ml-Dreihalskolben, ausgestattet mit elektrischem Heizmantel, Rührer, Tropftrichter und Thermometer, wurden 50 g Glutaminsäure unter Überleiten von Stickstoff für eine halbe Stunde auf 180 °C erhitzt. Bei der Reaktionsapparatur handelte es sich um eine offene Apparatur, so daß das während der Reaktion entstandene Wasser durch den Stickstoffstrom zum großen Teil entfernt wurde. Man kühlte die Schmelze auf 120 °C ab und tropfte während einer halben Stunde 11,1 g (10 Gew.-% bezogen auf Gesamtansatz) eines kammartigen Aminopropylpolydimethylsiloxans (3,8 % Stickstoff) zu. Man erhitzte auf 170 °C und gab dann während einer halben Stunde 50 g Asparaginsäure zu. Nach 1 h bei 170 °C goß man die noch heiße Schmelze in einen Mörser aus. Nachdem die Schmelze zu einer glasartigen Masse erstarrt war, wurde sie möglichst fein gemörsert. Das gelbe Pulver (61 g) wurde zunächst mit 1 l 0,1 N Natronlauge, dann mit 400 ml 1 N Natronlauge gerührt, bis eine fast klare Lösung entstand. Man neutralisierte mit 3 N wäßriger Salzsäure und destillierte anschließend das Wasser bei 100 °C im Ölpumpenvakuum ab. Man erhielt ein gelbes Pulver, dessen 1 %ige wäßrige Lösung opak war und gut schäumte.

### Anwendungstechnischer Teil:

### A) Physikalische Eigenschaften

Siliciumgehalt der Verbindungen aus den Beispielen 2 - 6:

| Verbindung aus Beispiel | Abkürzung¹⁾ | Siliciumgehalt [%] | Oberflächenspannung (0,1 % in Wasser) [mN/m] |
|---|---|---|---|
| 2 | Si-Pep (10 % A-Si) | 3 | n.b. |
| 3 | Si-Pep (20 % A-Si) | 6 | n.b. |
| 4 | Si-Pep (30 % A-Si) | 8 | 32,6 |
| 5 | Si-Pep (50 % A-Si) | 12 | n.b. |
| Vgl.-Beispiel | | <0,01 | 57,5²⁾ |

| | | | |
|---|---|---|---|
| n.b. = nicht bestimmt 1) Si-Pep = Siliconpeptid; A-Si = Aminofunktionelles Siloxan | | | |
| 2) Zum Vergleich: Die Oberflächenspannung von reinem Wasser beträgt 72 mN/m. | | | |

Die Tabelle verdeutlicht, daß durch die Menge des eingesetzten aminofunktionellen Siloxans der Siliconanteil im Polypeptid-Polysiloxan-Copolymer gezielt eingestellt werden kann. Ferner zeigt die Tabelle am Beispiel der Verbindung 4, daß die Siliconpeptide grenzflächenaktiv sind, da sie die Oberflächenspannung von Wasser (72 mN/m) deutlich reduzieren. Ein thermisches Protein zeigt zwar auch Grenzflächenaktivität, jedoch nicht in diesem hohen Maße.

### B) Sensorischer Haarsträhnchentest

Mit den Verbindungen aus den Beispielen 2 - 5 wurde an standardisiert vorgeschädigten 2 g schweren Euro-Haarbündeln ein Haarsträhnchentest durchgeführt. Dabei wurden die Haare standardisiert mit einer wäßrigen Shampooformulierung behandelt, die neben 9 % Natriumlaurylethersulfat und 3 % Cocoamidopropylbetain 1 % (Aktivgehalt) an Konditioniermittel enthielt. Die Lösungen waren ferner mit Natriumchlorid verdickt und die pH-Werte auf ca. 5,5 eingestellt. Zum Vergleich wurde ein im Markt bekanntes Protein-Silicon-Copolymer (Crodasone® W; EP-A-0 540 357) sowie eine Blindprobe (ohne Konditioniermittel = Placebo) herangezogen. Der Sensoriktest wurde von sechs ausgewählten Prüfpersonen als Rangordnungsprüfung, innerhalb der eine Differenzierung erzwungen wird, durchgeführt.

Die Haarsträhnchen wurden bezüglich ihrer Trockeneigenschaften (Trockenkämmbarkeit, Trockengriff und Glanz) und Naßeigenschaften (Entknotung, Naßkämmbarkeit, Naßgriff) beurteilt. Die Ergebnisse sind in den Tabellen 1 und 2 dargestellt.

Bei der Trockenkämmbarkeit ist eine Differenzierung gegenüber Placebo und Marktprodukt deutlich, es ist sogar eine Abstufung nach fallendem Silicongehalt erkennbar. Beim Trockengriff ist keine Differenzierung möglich, beim Glanz streuen die Ergebnisse. Auffällig ist, daß das Marktprodukt nicht besser als das Placebo ist.

Bei den Naßeigenschaften sticht bei der Entknotung und bei der Naßkämmbarkeit das auf 30 % Aminosiloxan basierende Produkt hervor; bei der Naßkämmbarkeit wurde auch das auf 50 % Aminosiloxan basierende Produkt gut beurteilt. Beim Naßgriff haben alle vier Produkte gut abgeschnitten. Bei den Naßeigenschaften ist eine Differenzierung gegenüber Placebo erkennbar.

Zusammenfassend ist festzuhalten, daß die konditionierenden Eigenschaften der Siliconpeptide aus den erfindungsgemäßen Beispielen 2 bis 5 - besonders bei den Trockeneigenschaften - deutlich sind. Die Produkte mit den höheren Siliconanteilen sind dabei am besten. Das Marktprodukt zeigte dagegen keine konditionierenden Eigenschaften.

**Tabelle 1**

| Haarsträhnchentest: Trockeneigenschaften | | | |
|---|---|---|---|
| Produkt | Trockenkämmbarkeit¹⁾ | Trockengriff¹⁾ | Glanz¹⁾ |
| Si-Pep (10 % A-Si) | 44 | 63 | 69 |
| Si-Pep (20 % A-Si) | 63 | 58 | 43 |
| Si-Pep (30 % A-Si) | 69 | 58 | 58 |
| Si-Pep (50 % A-Si) | 72 | 58 | 75 |
| Crodasone W | 50 | 55 | 43 |
| Placebo | 50 | 55 | 58 |

| | | | |
|---|---|---|---|
| ¹⁾ Jeweils als Rangordnungssumme in % | | | |

**Tabelle 2**

| Haarsträhnchentest: Naßeigenschaften. | | | |
|---|---|---|---|
| Produkt | Entknotung¹⁾ | Naßkämmbarkeit¹⁾ | Naßgriff¹⁾ |
| Si-Pep (10 % A-Si) | 63 | 63 | 69 |
| Si-Pep (20 % A-Si) | 61 | 41 | 67 |
| Si-Pep (30 % A-Si) | 83 | 78 | 52 |
| Si-Pep (50 % A-Si) | 52 | 69 | 67 |
| Crodasone W | 36 | 39 | 41 |
| Placebo | 52 | 59 | 52 |

| | | | |
|---|---|---|---|
| ¹⁾ Jeweils als Rangordnungssumme in % | | | |

## Patentansprüche

1. Polypeptid-Polysiloxan-Copolymere, bestehend aus wenigstens einer Polysiloxaneinheit 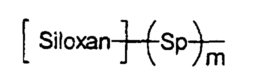 wobei der Index m eine positive ganze Zahl im Bereich von m = 1 - 52 darstellt,
der allgemeinen durchschnittlichen Formel I: 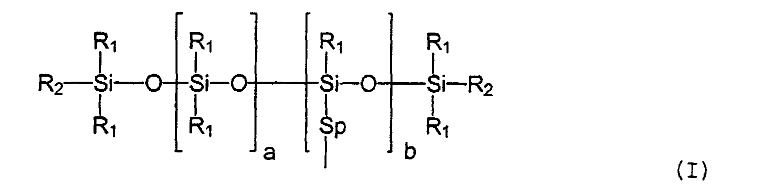 mit
R₁ = Alkylrest mit 1 bis 4 C-Atomen,
R₂ = R₁ und/oder ― Sp ―
wobei
- Sp - = divalenter Abstandshalter (Spacer) zwischen Siloxan und einer weiteren funktionellen Gruppe ist, wobei Siliciumatom und Abstandshalter über eine Silicium-Kohlenstoff-Bindung verknüpft sind, insbesondere ein divalenter Alkylenrest mit 1 bis 20 C-Atomen, der gegebenenfalls verzweigt ist, Doppelbindungen oder aromatische Ringe sowie Heteroatome, insbesondere Sauerstoff, Stickstoff oder Schwefel enthält,
die Indices a und b ganze Zahlen in den Bereichen von a = 0 - 200 und b = 0 - 50 darstellen,
mit der Maßgabe, daß für a = b = 0 und für b = 0 und a ≠ 0 jeweils wenigstens ein R₂ = - Sp - ist
und aus wenigstens einer Polypeptideinheit 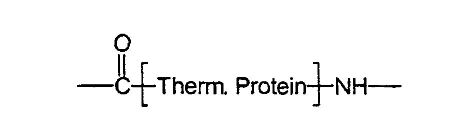 wobei
Therm. Protein für eine Struktur
der allgemeinen durchschnittlichen Formel II: 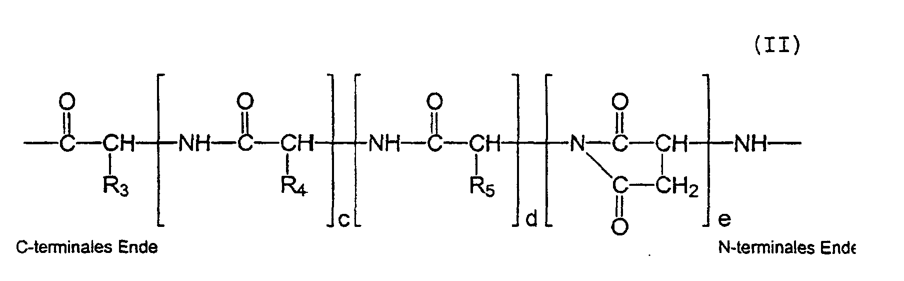 oder der Formel III: 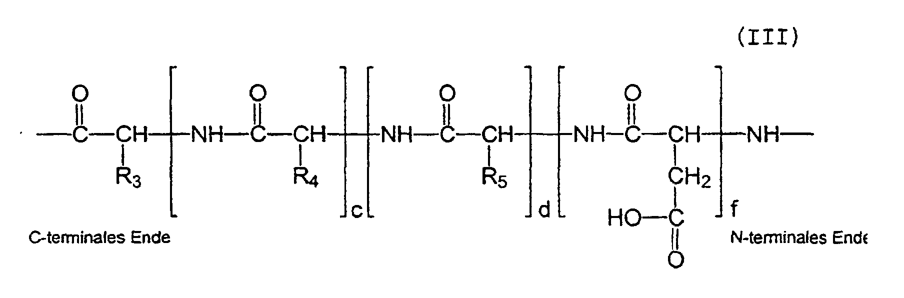 steht,
die über eine divalente funktionelle Gruppe
entweder über das C-terminale, das N-terminale Ende oder beide Enden der Polypeptideinheit mit der Polysiloxaneinheit verknüpft ist und für eine Struktureinheit - CH(OH)CH₂- oder -CH(OH)CH₂O-, -CO-, -CH(CH₂CO₂H)CO-, -NH-, -O-, -S-,-CH(NH₂)CO- oder -CH(CO₂H)NH-
steht
und gegebenenfalls über die Reste R₄ und/oder R₅ zusätzliche Verknüpfungen zwischen Polysiloxan- und Polypeptideinheiten erfolgen
mit
R₃ = R₄ oder R₅,
wobei
R₄ = gleich einem Rest einer Aminosäure sowie -(CH₂)₄-NH-R₆ ist,
mit R₆ = H (Lysin) oder 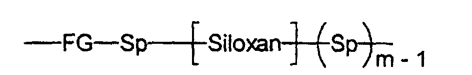 R₅ = -CH₂-CH₂-CO-R₆
mit R₆ = OH (Glutaminsäure) oder 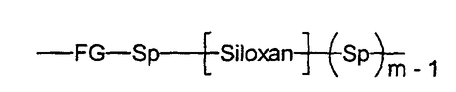 c, d, e und f positive ganze Zahlen einschließlich 0 sind, mit den Maßgaben, daß
die Indices c, d, e in Formel II und c, d und f in Formel III nicht sämtlich 0 sind,
insbesondere e ≠ 0, wenn c = 0 ist,
c und d ≠ 0 sind, wenn e oder f = 0 ist
sowie
die Molekularmasse der Polypeptideinheit zwischen 250 und 9 000 liegt
und das Gewichtsverhältnis von Polysiloxaneinheiten und Polypeptideinheiten im Polypeptid-Polysiloxan-Copolymer zwischen 1 : 99 und 99 : 1 liegt.

2. Polypeptid-Polysiloxan-Copolymere gemäß Anspruch 1, **dadurch gekennzeichnet, daß**
R₁ = CH₃, m = 2 - 32, a = 8 - 100, b = 0 - 30 ist, wobei für b = 0 beide Reste R₂ dann - Sp - entsprechen.

3. Polypeptid-Polysiloxan-Copolymere gemäß Anspruch 1 oder 2, **dadurch gekennzeichnet, daß** m = 2 - 17, a = 8 - 40, b = 0 - 15 ist, wobei für b = 0 beide Reste R₂ dann - Sp - entsprechen.

4. Polypeptid-Polysiloxan-Copolymere gemäß einem der Ansprüche 1 bis 3, **dadurch gekennzeichnet, daß**
c, d und e in Formel II oder c, d und f in Formel III ≠ 0 sind, wobei
das Gewichtsverhältnis von Polysiloxaneinheiten und Polypeptideinheiten im Polypeptid-Polysiloxan-Copolymer zwischen 5 : 95 und 55 : 45 liegt.

5. Polypeptid-Polysiloxan-Copolymere gemäß einem der Ansprüche 1 bis 4, **dadurch gekennzeichnet, daß**
c in Formel II oder Formel III = 0 und d und e in Formel II oder d und f in Formel III ≠ 0 sind
und R₃ = R₅ oder CH₂-CO-R₆
mit R₆ = OH (Asparaginsäure) oder 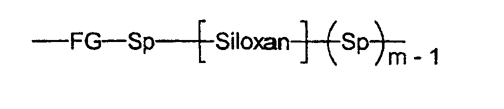 und das Gewichtsverhältnis von Polysiloxaneinheiten und Polypeptideinheiten im Polypeptid-Polysiloxan-Copolymer zwischen 5 : 95 und 55 : 45 liegt.

6. Polypeptid-Polysiloxan-Copolymere gemäß einem der Ansprüche 1 bis 3, **dadurch gekennzeichnet, daß**
c und d in Formel II oder Formel III = 0 sind und e oder f in Formel II oder Formel III ≠ 0 sind
und R₃ = -CH₂-CO-R₆
mit R₆ = OH (Asparaginsäure) oder 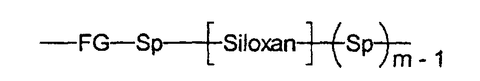 und das Gewichtsverhältnis von Polysiloxaneinheiten und Polypeptideinheiten im Polypeptid-Polysiloxan-Copolymer zwischen 5 : 95 und 55 : 45 liegt.

7. Polypeptid-Polysiloxan-Copolymere gemäß Anspruch 1, **dadurch gekennzeichnet, daß**
-Sp- ausgewählt ist aus
- (CH₂)₃-, -(CH₂)₃-O-CH₂- oder -(CH₂)₃-NH - (CH₂)₂-
und
-FG- ausgewählt ist aus:
- CH(OH)CH₂- oder -CH(OH)CH₂O-, -CO-, -CH(CH₂CO₂H)CO-,-NH-,
-O-, -S-,-CH(NH₂)CO- oder -CH(CO₂H)NH-.

8. Polypeptid-Polysiloxan-Copolymere gemäß Anspruch 1, **dadurch gekennzeichnet, daß**
-Sp- ausgewählt ist aus
- (CH₂)₃- oder -(CH₂)₃-NH-(CH₂)₂-
und
-FG- = -NH- ist.

9. Polypeptid-Polysiloxan-Copolymere gemäß einem der Ansprüche 1 bis 8, **dadurch gekennzeichnet, daß** die Aminosäure ausgewählt ist aus Glycin, Alanin, Valin, Leucin, Isoleucin, Phenylalanin, Prolin, Serin, Threonin, Tyrosin, Asparagin, Glutamin, Arginin, Tryptophan, Histidin, Cystein, Methionin, Asparaginsäure und Glutaminsäure.

10. Verfahren zur Herstellung von Polypeptid-Polysiloxan-Copolymeren gemäß einem der Ansprüche 1 bis 9 durch thermische Polymerisation von Aminosäuren der allgemeinen Formel: 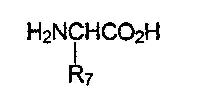 wobei R₇ gleich oder verschieden der Rest einer Aminosäure wie Glycin, Alanin, Valin, Leucin, Isoleucin, Phenylalanin, Prolin, Serin, Threonin, Tyrosin, Asparagin, Glutamin, Arginin, Lysin, Tryptophan, Histidin, Cystein, Methionin, Asparaginsäure, Glutaminsäure ist, in Anwesenheit von Organopolysiloxanen mit reaktiven Gruppen
- RG-
der allgemeinen durchschnittlichen Formel (I') 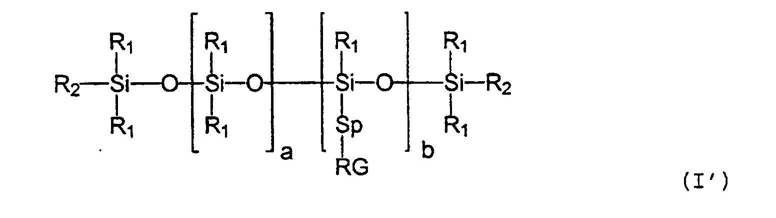 mit den Maßgaben, daß
im Mittel wenigstens ein Rest ― Sp ― RG enthalten ist, insbesondere für b = 0 wenigstens einer der beiden Reste R₂ dann einem Rest
-Sp - RG entspricht,
wobei RG eine Epoxy-, Carboxy-, Amino-, Thio-, Aminosäure-, oder Hydroxygruppe ist
und gegebenenfalls zusätzlich durch alkalische Hydrolyse der Succinimideinheiten im Polypeptid zu Asparaginsäureeinheiten.

11. Verfahren gemäß Anspruch 10, **gekennzeichnet durch** ein Gemisch der Aminosäuren Asparaginsäure, Glutaminsäure und einer oder mehreren weiteren Aminosäuren.

12. Verfahren gemäß Anspruch 10, **gekennzeichnet durch** ein Gemisch der Aminosäuren Asparaginsäure und Glutaminsäure.

13. Verfahren gemäß Anspruch 10, **dadurch gekennzeichnet, daß** die Aminosäure ausschließlich Asparaginsäure ist.

14. Verfahren gemäß einem der Ansprüche 11 bis 13, **dadurch gekennzeichnet, daß** das Gewichtsverhältnis von Asparaginsäure zu Glutaminsäure zwischen 5 : 1 und 1 : 5 liegt und der Anteil weiterer Aminosäuren am Gemisch 0 bis 30 Gew.-% beträgt.

15. Verwendung der Verbindungen gemäß einem der Ansprüche 1 bis 9 als grenzflächenaktive oder Affinität zu Oberflächen aufweisende Substanzen, insbesondere in polaren Medien.

16. Verwendung gemäß Anspruch 15 in kosmetischen Zubereitungen als Tenside zur Reinigung, Pflege oder Konditionierung von Haut und Haar, als Öl-in-Wasser-, Wasser-in-Öl-Emulgatoren oder als Stabilisatoren in kosmetischen Emulsionen.

17. Verwendung gemäß Anspruch 15 als Tenside in technischen Anwendungen als Hilfsmittel zur Verbesserung der Eigenschaften von Textilien, als Hilfsmittel zur Verbesserung der Eigenschaften von Farben und Lacken oder als Hilfsmittel zur Verbesserung der Eigenschaften von Kunststoffen.

## Claims

1. Polypeptide-polysiloxane copolymers consisting of at least one polysiloxane unit 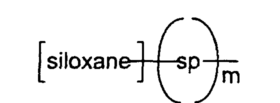 where the index m is a positive integer in the range m = 1 - 52,
of the general average formula I: 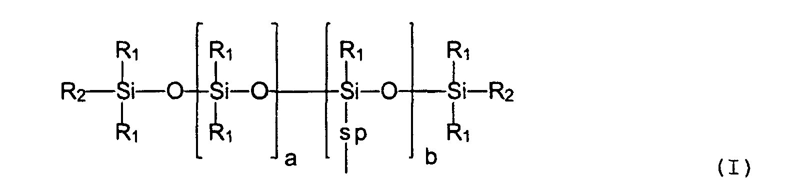 where
R₁ = alkyl radical having from 1 to 4 carbon atoms,
R₂ = R₁ and/or ― sp―
where
- sp - = divalent spacer between siloxane and another functional group, silicon atom and spacer being linked via a silicon-carbon bond, in particular a divalent alkylene radical having from 1 to 20 carbon atoms, which is optionally branched, and may contain double bonds or aromatic rings, and heteroatoms, in particular oxygen, nitrogen or sulphur,
the indices a and b are integers in the ranges a = 0 - 200 and b = 0 - 50,
with the proviso that when a = b = 0 and when b = 0 and a ≠ 0, at least one R₂ = - sp - in each case, and of at least one polypeptide unit 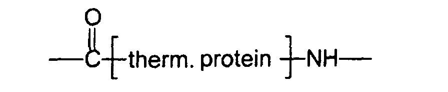 where
therm. protein is a structure of the general average formula II: 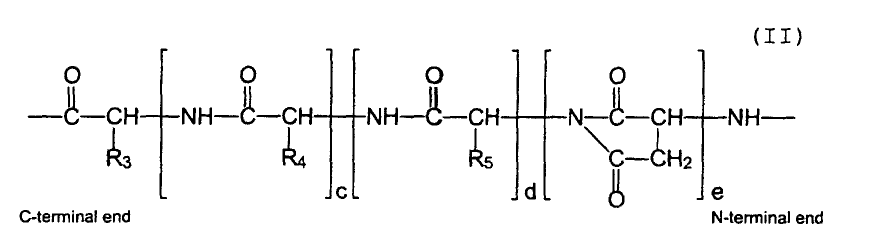 or of the formula III: 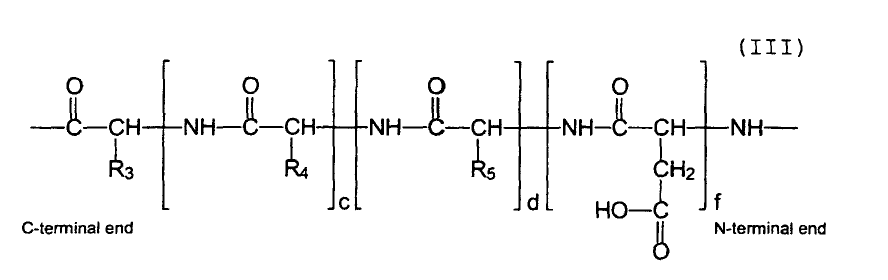 which is linked
to the polysiloxane unit via a divalent functional group
- FG -
either via the C-terminal end, the N-terminal end or both ends of the polypeptide unit, and is a structural unit
- CH(OH)CH₂- or -CH (OH) CH₂O-, -CO-, -CH (CH₂CO₂H) CO-, - NH-, -O-, -S-, -CH(NH₂)CO- or -CH(CO₂H)NH-
and optionally additional links between polysiloxane and polypeptide units result via the radicals R₄ and/or R₅
where
R₃ = R₄ or R₅,
where
R₄ = is identical to a residue of an amino acid and - (CH₂)₄-NH-R₆,
where R₆ = H (lysine) or 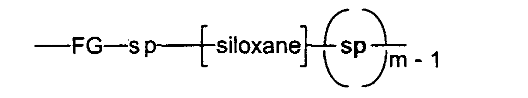 R₅ = -CH₂-CH₂-CO-R₆
where R₆ = OH (glutamic acid) or 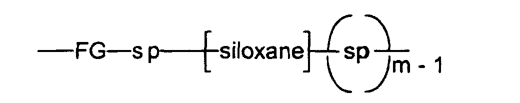 c, d, e and f are positive integers including 0, with the provisos that
the indices c, d, e in formula II and c, d and f in formula III are not all 0,
in particular e ≠ 0, when c = 0,
c and d ≠ 0, when e or f = 0,
and
the molecular mass of the polypeptide unit is between 250 and 9000
and the weight ratio of polysiloxane units and polypeptide units in the polypeptide-polysiloxane copolymer is between 1 : 99 and 99 : 1.

2. Polypeptide-polysiloxane copolymers according to Claim 1, **characterized in that**
R₁ = CH₃, m = 2 - 32, a = 8 - 100, b = 0 - 30,
where, when b = 0, both radicals R₂ then correspond to - sp -.

3. Polypeptide-polysiloxane copolymers according to Claim 1 or 2, **characterized in that** m = 2 - 17, a = 8 - 40, b = 0 - 15, where, when b = 0, both radicals R₂ then correspond to - sp -.

4. Polypeptide-polysiloxane copolymers according to one of Claims 1 to 3, **characterized in that**
c, d and e in formula II or c, d and f in formula III ≠ 0, where
the weight ratio of polysiloxane units and polypeptide units in the polypeptide-polysiloxane copolymer is between 5 : 95 and 55 : 45.

5. Polypeptide-polysiloxane copolymers according to one of Claims 1 to 4, **characterized in that**
c in formula II or formula III = 0 and d and e in formula II or d and f in formula III ≠ 0
and R₃ = R₅ or -CH₂-CO-R₆
where R₆ = OH (aspartic acid) or 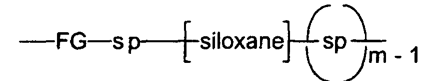 and the weight ratio of polysiloxane units and polypeptide units in the polypeptide-polysiloxane copolymer is between 5 : 95 and 55 : 45.

6. Polypeptide-polysiloxane copolymers according to one of Claims 1 to 3, **characterized in that**
c and d in formula II or formula III = 0, and e or f in formula II or formula III ≠ 0
and R₃ = -CH₂-CO-R₆
where R₆ = OH (aspartic acid) or 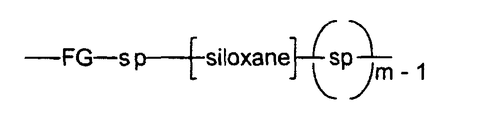 and the weight ratio of polysiloxane units and polypeptide units in the polypeptide-polysiloxane copolymer is between 5 : 95 and 55 : 45.

7. Polypeptide-polysiloxane copolymers according to Claim 1, **characterized in that**
- sp- is chosen from
- (CH₂)₃-, -(CH₂)₃-O-CH₂- or - (CH₂) ₃-NH- (CH₂) ₂-
and
- FG- is chosen from:
- CH(OH)CH₂- or -CH(OH)CH₂O-, -CO-, -CH(CH₂CO₂H)CO-, - NH-, -O-, -S-, -CH(NH₂)CO- or -CH(CO₂H)NH-.

8. Polypeptide-polysiloxane copolymers according to Claim 1, **characterized in that**
- sp- is chosen from
- (CH₂) ₃- or -(CH₂)₃-NH-(CH₂)₂-
and
- FG- = -NH-.

9. Polypeptide-polysiloxane copolymers according to one of Claims 1 to 8, **characterized in that** the amino acid is chosen from glycine, alanine, valine, leucine, isoleucine, phenylalanine, proline, serine, threonine, tyrosine, asparagine, glutamine, arginine, tryptophan, histidine, cysteine, methionine, aspartic acid and glutamic acid.

10. Process for the preparation of polypeptide-polysiloxane copolymers according to one of Claims 1 to 9 by thermal polymerization of amino acids of the general formula: 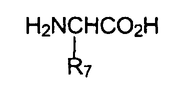 where R₇ is identical or different and is the radical of an amino acid such as glycine, alanine, valine, leucine, isoleucine, phenylalanine, proline, serine, threonine, tyrosine, asparagine, glutamine, arginine, lysine, tryptophan, histidine, cysteine, methionine, aspartic acid, glutamic acid, in the presence of organopolysiloxanes having reactive groups
- RG-
of the general average formula (I') 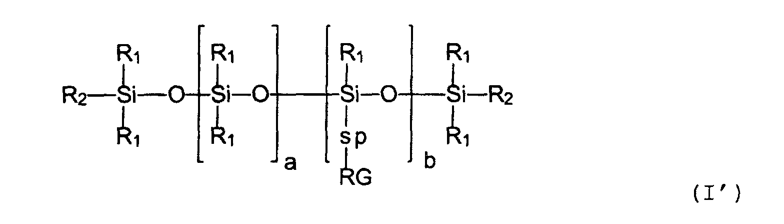 with the provisos that
on average at least one residue ― sp ― RG is present,
in particular when b = 0 at least one of the two radicals R₂ then corresponds to a residue
- sp - RG,
where RG is chosen from epoxy, carboxy, amino, thio, amino acid or hydroxyl groups and, where appropriate, additionally by alkaline hydrolysis of the succinimide units in the polypeptide to aspartic acid units.

11. Process according to Claim 10, **characterized by** a mixture of the amino acids aspartic acid, glutamic acid and one or more other amino acids.

12. Process according to Claim 10, **characterized by** a mixture of the amino acids aspartic acid and glutamic acid.

13. Process according to Claim 10, **characterized in that** the amino acid is exclusively aspartic acid.

14. Process according to one of Claims 11 to 13, **characterized in that** the weight raito of aspartic acid to glutamic acid is between 5 : 1 and 1 : 5, and the content of other amino acids in the mixture is from 0 to 30% by weight.

15. Use of the compounds according to one of Claims 1 to 9 as interface-active substances or substances having an affinity to surfaces, in particular in polar media.

16. Use according to Claim 15 in cosmetic preparations as surfactants for the cleaning, care or conditioning of skin and hair, as oil-in-water emulsifiers, water-in-oil emulsifiers or as stabilizers in cosmetic emulsions.

17. Use according to Claim 15 as surfactants in technical applications as auxiliaries for improving the properties of textiles, as auxiliaries for improving the properties of paints and varnishes or as auxiliaries for improving the properties of plastics.

## Revendications

1. Copolymères polypeptide/polysiloxane, constitués d'au moins une unité polysiloxane 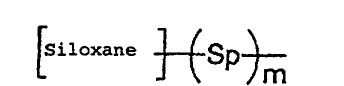 l'indice m représentant un nombre entier positif dans la plage de m = 1 - 52,
de formule moyenne générale I : 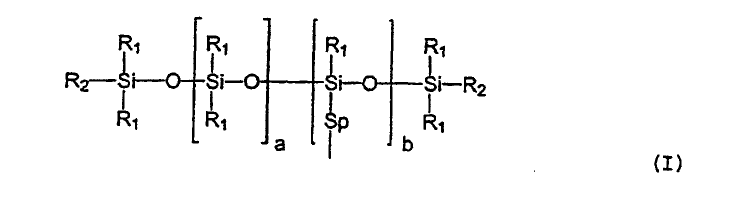 où
R₁ représente un radical alkyle ayant de 1 à 4 atomes de carbone,
R₂ = R₁ et/ou -Sp-
- Sp- étant un espaceur divalent entre le siloxane et un autre groupe fonctionnel, l'atome de silicium et l'espaceur étant reliés par une liaison silicium-carbone, en particulier un radical alkylène divalent ayant de 1 à 20 atomes de carbone, qui est éventuellement ramifié, comporte des doubles liaisons ou des noyaux aromatiques ainsi que des hétéroatomes, en particulier d'oxygène, d'azote ou de soufre, les indices a et b représentent des nombres entiers dans les plages de a = 0 - 200 et b = 0 - 50,
étant entendu que pour a = b = 0 et pour b =0 et a ≠ 0 chaque fois au moins un radical R₂ est -Sp- et d'au moins une unité polypeptide 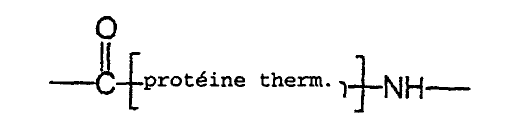 dans laquelle
protéine therm. représente une structure de formule moyenne générale II : 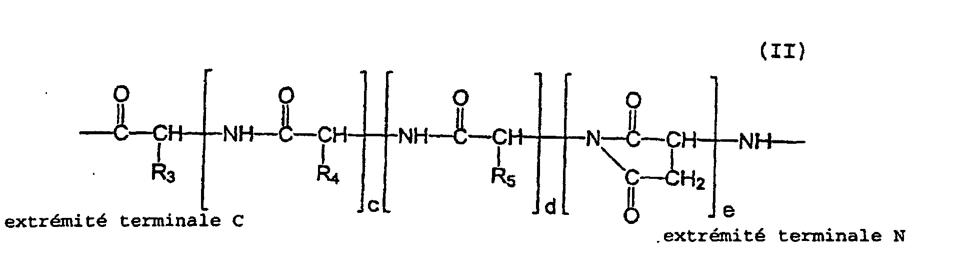 ou de formule III: 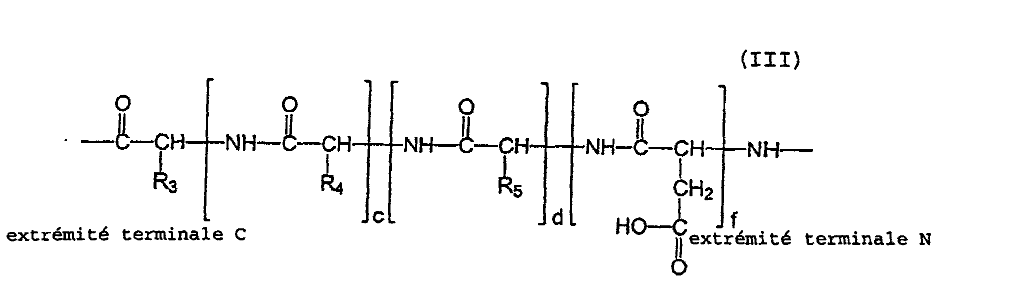 qui est reliée au moyen d'un groupe fonctionnel divalent
- FG -
soit par l'extrémité C-terminale, l'extrémité N-terminale ou les deux extrémités de l'unité polypeptide à l'unité polysiloxane et représente une unité structurale
- CH(OH)CH₂- ou -CH(OH)CH₂O-, -CO-, -CH(CH₂CO₂H)CO-, - NH-, -O-, -S-, -CH(NH₂)CO- ou -CH(CO₂H)NH-
et éventuellement des liaisons supplémentaires entre des unités polysiloxane et polypeptide s'effectuant par les radicaux R₄ et/ou R₅
où
R₃ = R₄ ou R₅,
R₄ représentant un reste d'un aminoacide ainsi que - (CH₂) ₄-NH-R₆,
où R₆ = H (lysine) ou 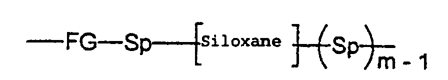 R₅ = -CH₂-CH₂-CO-R₆
où R₆ = OH (acide glutamique) ou 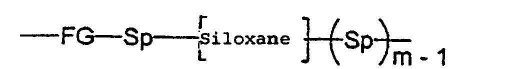 c, d, e et f sont des nombres entiers positifs 0 inclus,
étant entendu que
les indices c, d, e dans la formule II et les indices c, d et f dans la formule III ne sont pas tous 0,
en particulier e est ≠ 0 lorsque c est 0,
c et d sont ≠ 0 lorsque e ou f est 0,
et
la masse moléculaire de l'unité polypeptide est comprise entre 250 et 9 000
et le rapport pondéral des unités polysiloxane et des unités polypeptide dans le copolymère polypeptide-polysiloxane est compris entre 1:99 et 99:1.

2. Copolymères polypeptide-polysiloxane selon la revendication 1, **caractérisés en ce que**
R₁ = CH₃, m = 2 - 32, a = 8 - 100, b = 0 - 30, pour b = 0 les deux radicaux R₂ correspondant alors à -Sp-

3. Copolymères polypeptide-polysiloxane selon la revendication 1 ou 2, **caractérisés en ce que** m = 2 - 17, a = 8 - 40, b = 0 - 15, pour b = 0, les deux radicaux R₂ correspondant alors à -Sp-.

4. Copolymères polypeptide-polysiloxane selon l'une quelconque des revendications 1 à 3, **caractérisés en ce que**
c, d et e dans la formule II ou c, d et f dans la formule III sont ≠ 0,
le rapport pondéral des unités polysiloxane et des unités polypeptide dans le copolymère polypeptide-polysiloxane étant compris entre 5:95 et 55:45.

5. Copolymères polypeptide-polysiloxane selon l'une quelconque des revendications 1 à 4, **caractérisés en ce que**
c dans la formule II ou la formule III = 0 et d et e dans la formule II ou d et f dans la formule III sont ≠ 0
et R₃ = R₅ ou -CH₂-CO-R₆
où R₆ = OH (acide aspartique) ou 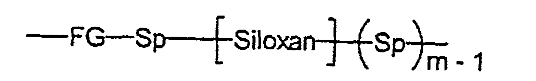 et le rapport, pondéral des unités polysiloxane et des unités polypeptide dans le copolymère polypeptide-polysiloxane est compris entre 5:95 et 55:45.

6. Copolymères polypeptide-polysiloxane selon l'une quelconque des revendications 1 à 3, **caractérisés en ce que**
c et d dans la formule II ou la formule III sont = 0 et e ou f dans la formule II ou la formule III est ≠ 0
et R₃ = -CH₂-CO-R₆
où R₆ = OH (acide aspartique) ou 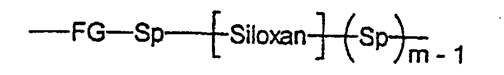 et le rapport pondéral des unités polysiloxane et des unités polypeptide dans le copolymère polypeptide-polysiloxane est compris entre 5:95 et 55:45.

7. Copolymères polypeptide-polysiloxane selon la revendication 1, **caractérisés en ce que**
- Sp- est choisi parmi
- (CH₂)₃-, -(CH₂)₃-O-CH₂- et -(CH₂)₃-NH-(CH₂)₂-
et
- FG- est choisi parmi :
- CH(OH)CH₂- ou -CH(OH)CH₂O-, -CO-, -CH(CH₂CO₂H)CO-, - NH-, -O-, -S-, -CH(NH₂)CO- et -CH(CO₂H)NH-.

8. Copolymères polypeptide-polysiloxane selon la revendication 1, **caractérisés en ce que**
- Sp- est choisi parmi
- (CH₂)₃- et -(CH₂)₃-NH-(CH₂)₂-
et
- FG- est -NH-.

9. Copolymères polypeptide-polysiloxane selon l'une quelconque des revendications 1 à 8, **caractérisés en ce que** l'aminoacide est choisi parmi la glycine, l'alanine, la valine, la leucine, l'isoleucine, la phénylalanine, la proline, la sérine, la thréonine, la tyrosine, l'asparagine, la glutamine, l'arginine, le tryptophane, l'histidine, la cystéine, la méthionine, l'acide aspartique et l'acide glutamique.

10. Procédé pour la préparation de copolymères polypeptide-polysiloxane selon l'une quelconque des revendications 1 à 9, par polymérisation thermique d'aminoacides de formule générale : 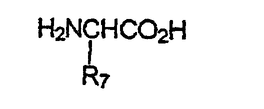 dans laquelle les radicaux R₇ sont identiques ou différents et représentent le reste d'un aminoacide tel que la glycine, l'alanine, la valine, la leucine, l'isoleucine, la phénylalanine, la proline, la sérine, la thréonine, la tyrosine, l'asparagine, la glutamine, l'arginine, la lysine, le tryptophane, l'histidine, la cystéine, la méthionine, l'acide aspartique, l'acide glutamique, en présence d'organopolysiloxanes comportant des groupes réactifs
- RG-
de formule moyenne générale (I') 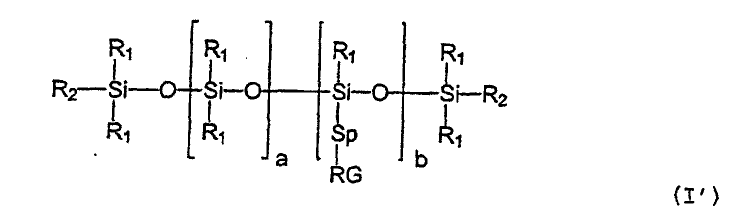 étant entendu
qu'au moins au milieu un radical -Sp-RG est contenu,
en particulier pour b = 0 au moins l'un des deux radicaux R₂ correspond alors à un radical
- Sp-RG,
RG étant un groupe époxy, carboxy, amino, thio, aminoacide ou hydroxy
et éventuellement en outre par hydrolyse alcaline des restes succinimide dans le polypeptide en restes acide aspartique.

11. Procédé selon la revendication 10, **caractérisé par** un mélange des aminoacides acide aspartique, acide glutamique et d'un ou plusieurs autres aminoacides.

12. Procédé selon la revendication 10, **caractérisé par** un mélange des aminoacides acide aspartique et acide glutamique.

13. Procédé selon la revendication 10, **caractérisé en ce que** l'aminoacide est exclusivement l'acide aspartique.

14. Procédé selon l'une quelconque des revendications 11 à 13, **caractérisé en ce que** le rapport pondéral de l'acide aspartique à l'acide glutamique est compris entre 5:1 et 1:5, et la proportion d'autres aminoacides dans le mélange va de 0 à 30 % en poids.

15. Utilisation des composés selon l'une quelconque des revendications 1 à 9, en tant que substances tensioactives ou présentant une affinité pour des surfaces, en particulier dans des milieux polaires.

16. Utilisation selon la revendication 15 dans des préparations cosmétiques en tant que tensioactifs pour le nettoyage, le soin ou le conditionnement de la peau et des cheveux, en tant qu'émulsifiants huile-dans-eau, eau-dans-huile ou en tant que stabilisants dans des émulsions cosmétiques.

17. Utilisation selon la revendication 15, en tant que tensioactifs dans des applications industrielles en tant qu'adjuvants destinés à améliorer les caractéristiques de textiles, en tant qu'adjuvants destinés à améliorer les caractéristiques de couleurs et de peintures ou en tant qu'adjuvants destinés à améliorer les propriétés de matières plastiques.
